# EUROPÄISCHE PATENTSCHRIFT

(11) **EP 2 143 779 B1**
(45) Veröffentlichungstag und Bekanntmachung des Hinweises auf die Patenterteilung: **22.09.2010**
(21) Anmeldenummer: 09005892.6
(22) Anmeldetag: 28.04.2009
(51) Int. Cl.: C10L 3/00, F17C 13/02

(54) **Verfahren zum kontrollierten Zumischen eines Odoriermediums**
Method for controlled mixing of an odorising medium
Procédé de mélange contrôlé d'un milieu odorant

(30) Priorität: 08.07.2008 DE 102008032108
(43) Veröffentlichungstag der Anmeldung: 13.01.2010
(73) Patentinhaber: Linde AG, 80331 München (DE)
(72) Erfinder: Schön, Helmut, Dr., 85276 Pfaffenhofen (DE); Stoppa, Heinz-Peter, 85778 Haimhausen (DE)
(74) Vertreter: Zahn, Christoph

(56) Entgegenhaltungen:
- US-A- 3 634 053
- US-A- 4 007 755
- US-A- 5 143 257
- US-B1- 6 208 913

## Beschreibung

Die Erfindung betrifft ein Verfahren zum kontrollierten Zumischen eines Odoriermediums zu einem Medium, insbesondere zu Kohlendioxid, wobei das Odoriermedium und das Medium in einem bzgl. seines Volumens veränderbaren Dosierbehälter vermischt werden. Ein derartiges Verfahren ist z.B. der Druckschriff US-A-3 634 053 zu entnehmen.

Unter dem Begriff "Medium" seien nachfolgend Flüssigkeiten, einschließlich unter Druck verflüssigter Gase, sowie Gase zu verstehen. Gattungsgemäße Verfahren zum kontrollierten Zumischen eines Odoriermediums zu einem Medium, wie Kohlendioxid, werden beispielsweise seitens der Kraftfahrzeug-Industrie gewünscht, um Kohlendioxid, das als Wärmetransportmittel in Klimaanlagen Verwendung findet, odorieren zu können. Mittels dieses Odorierens können die Fahrzeuginsassen einen Austritt des geruchslosen Kohlendioxids aus defekten Klimaanlagen wahrnehmen. Als Odorierungsmedium kommen insbesondere geruchsintensive Flüssigkeiten, wie beispielsweise Gasodor S-Free D611838 (ein Gemisch, bestehend aus Ethylacrylat, Methylacrylat und 2-Ethyl-3-Methylpyrazin) der Fa. Symrise AG, zur Anwendung. Pro kg Kohlendioxid beträgt die Menge des Odoriermediums etwa 30 bis 1000 mg.

Aufgabe der vorliegenden Erfindung ist es, ein gattungsgemäßes Verfahren zum kontrollierten Zumischen eines Odoriermediums zu einem Medium anzugeben, das ein verfahrenstechnisch einfaches und dabei exaktes Vermischen des Odoriermediums mit dem Medium ermöglicht.

Zur Lösung dieser Aufgabe wird ein Verfahren zum kontrollierten Zumischen eines Odoriermediums zu einem Medium vorgeschlagen, das folgende Verfahrensschritte umfasst:
a) Einstellen eines definierten Dosierbehälter-Volumens (2),
b) Evakuieren des Dosierbehälters (1),
c) Einleiten einer definierten Menge des Odoriermediums (12) in den Dosierbehälter (1), wobei das Einleiten der definierten Menge des Odoriermediums (12) so lange fortgesetzt wird, bis das Dosierbehälter-Volumen (2) vollständig ausgefüllt ist, und
d) Durchleiten einer definierten Menge des Mediums (4, 5, 6) durch das mit dem Odoriermedium (12) befüllten Dosierbehälter-Volumen (2).

Weitere vorteilhafte Ausgestaltungen des erfindungsgemäßen Verfahrens zum kontrollierten Zumischen eines Odoriermediums zu einem Medium, die Gegenstände der abhängigen Patentansprüche darstellen, sind dadurch gekennzeichnet, dass
- der Dosierbehälter (1) auf einen Druck zwischen 10 und 20 mbar evakuiert wird,
- das während des Verfahrensschrittes b) aus dem Dosierbehälter (1) abgezogene Gemisch adsorptiv (8) von dem Odoriermedium befreit wird, und
- das Medium Kohlendioxid ist.

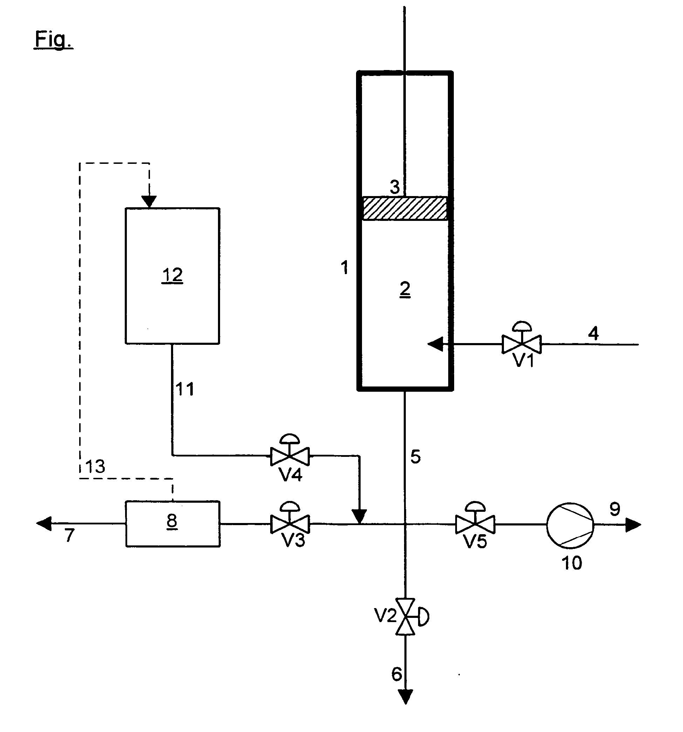

Das erfindungsgemäße Verfahren zum kontrollierten Zumischen eines Odoriermediums zu einem Medium sowie vorteilhafte Ausgestaltungen desselben seien nachfolgend anhand des in der Figur dargestellten Ausführungsbeispieles näher erläutert.

Die Figur zeigt einen Dosierbehälter 1, dessen Innenraum bzw. Volumen 2 verändert werden kann. Dies kann beispielsweise mittels eines Kolbens 3, der in dem zylinderformigen Dosierbehälter 1 verschiebbar angeordnet ist, geschehen. Alternativ hierzu kann ein in der Figur nicht dargestellter Faltenbalg vorgesehen werden, über den das Innenvolumen 2 des Dosierbehälters 1 variiert werden kann. Die Bewegung des Kolbens 3 bzw. eines Faltenbalges erfolgt vorzugsweise mittels eines Motorantnebes oder eines pneumatischen Stellgliedes.

Über die Leitung 4, in der ein Regelventil V1 angeordnet ist, wird dem Dosierbehälter 1 bzw. dessen Innenraum 2 das zu odorierende Medium zugeführt. Nach erfolgter Vermischung von Odoriermedium und dem zu odorierenden Medium wird das dabei entstehende Gemisch bei geöffnetem Regelventil V2 über die Leitungsabschnitte 5 und 6 abgezogen und beispielsweise einem in der Figur nicht dargestellten Druckgasbehälter zugeführt.

Ergänzend und/oder alternativ zu der vorbeschriebenen Zuführung des zu odorierenden Mediums über die Leitung 4, kann eine Einbringung des zu odorierenden Mediums (ergänzend) durch den Kolben 3 erfolgen.

Über die Leitungsabschnitte 5 und 9 kann bei geöffnetem Regelventil V5 mittels der Vakuumpumpe 10 ein Evakuieren des Dosierbehälters 1 realisiert werden.

Das Odoriermedium wird vorzugsweise in einem Speicherbehälter 12 bevorratet. Aus diesem kann es bei geöffnetem Regelventil V4 über die Leitungsabschnitte 11 und 5 in den Dosierbehälter 1 geführt werden.

Das erfindungsgemäße Verfahren zum kontrollierten Zumischen eines Odoriermediums zu einem zu odorierenden Medium wird realisiert, indem bei geschlossenen Ventilen V1, V2, V4 und V5 der Innenraum 2 des Dosierbehälters 1 über die Leitungsabschnitte 5 und 7 bei geöffnetem Regelventil V3 mit der Atmosphäre verbunden wird. Hierbei kann in dem Leitungsabschnitt 7 eine Adsorptionseinheit 8 vorgesehen werden, die der Entfernung des Odoriermediums aus dem den Innenraum 2 des Dosierbehälters 1 verlassenden Gasgemisches dient. Die Adsorptionseinheit 8 weist vorzugsweise ein oder mehrere Adsorptionsmittel auf, das bzw. die für die Entfernung des oder der Odoriermedien geeignet ist bzw. sind.

Das adsorptiv entfernte Odoriermedium kann nach einer ggf. vorzusehenden Nachreinigung über die gestrichelt gezeichnete Leitung 13 erneut dem Speicherbehälter 12 zugeführt werden.

Nach erfolgtem Druckausgleich zwischen dem Innenraum 2 des Dosierbehälters 1 und der Atmosphäre wird mittels des Kolbens 3 ein definiertes Innenvolumen 2 in dem Dosierbehälter 1 eingestellt.

Anschließend wird Ventil V3 geschlossen und Ventil V5 geöffnet, so dass über die Leitungsabschnitte 5 und 9 mittels der Vakuumpumpe 10 ein Evakuieren des zuvor eingestellten Dosierbehälter-Volumens 2 erfolgen kann. Hierbei wird der Dosierbehälter 1 vorzugsweise auf einen Druck zwischen 10 und 20 mbar evakuiert. Auch im Leitungsabschnitt 9 ist vorzugsweise ein in der Figur nicht dargestellte Adsorptionseinheit, die der Entfernung des oder der Odoriermedien dient, angeordnet.

Nach Erreichen des gewünschten Enddruckes innerhalb des Volumens 2 des Dosierbehälters 1 wird eine definierte Menge des Odoriermediums aus dem Speicherbehälter 12 bei geöffnetem Ventil 4 über die Leitungsabschnitte 11 und 5 in den Dosierbehälter 1 geführt bzw. entspannt. Hierbei wird das Odoriermedium so lange in den Dosierbehälter 1 geleitet, bis dessen Innenvolumen 2 vollständig ausgefüllt ist. Anschließend wird das Regelventil V4 geschlossen.

Sofern das in dem Speicherbehälter 12 bevorratete Odoriermedium eine hohe Toxizität aufweist, wird vorzugsweise mittels einer geeigneten Vorrichtung innerhalb des Speicherbehälters 12 eine inerte Atmosphäre - beispielsweise durch die Zugabe von Stickstoff oder eines Edelgases - geschaffen.

Als letzter Verfahrensschritt werden nunmehr ausschließlich die Ventile V1 und V2 geöffnet. Über die Leitung 4 wird sodann eine definierte Menge des zu odorierenden Mediums in bzw. durch den Dosierbehälter 1 geleitet. Das durch die Vermischung mit dem Odoriermedium entstehende Mediengemisch wird über die Leitungsabschnitte 5 und 6 abgezogen und, wie bereits erwähnt, beispielsweise einem Druckgasbehälter zugeführt. Nach erfolgter Entspannung des Innenraumes 2 des Dosierbehälters 1 über die Leitungsabschnitte 5 und 7, kann die vorstehend beschriebene Zumischprozedur erneut begonnen werden.

Die in der Figur dargestellte Ausgestaltung des Dosierbehälters 1 sollte in der Praxis eine Duckfestigkeit von wenigstens 200 bis 250 bar gewährleisten.

Das erfindungsgemäße Verfahren zum kontrollierten Zumischen eines Odoriermediums zu einem Medium ermöglicht nunmehr eine im Hinblick auf Menge und/oder Konzentration des Odoriermediums und/oder des zu odorierenden Mediums variable Betriebsweise. Auch können unterschiedliche Odoriermedien zur Anwendung kommen und - zeitgleich oder zeitlich versetzt - dem zu odorierenden Medium zugemischt werden.

## Patentansprüche

1. Verfahren zum kontrollierten Zumischen eines Odoriermediums zu einem Medium, wobei das Odoriermedium und das Medium in einem bzgl. seines Volumens (2) veränderbaren Dosierbehälter (1) vermischt werden, umfassend folgende Verfahrensschritte:
a) Einstellen eines definierten Dosierbehälter-Volumens (2),
b) Evakuieren des Dosierbehälters (1),
c) Einleiten einer definierten Menge des Odoriermediums (12) in den Dosierbehälter (1), wobei das Einleiten der definierten Menge des Odoriermediums (12) so lange fortgesetzt wird, bis das Dosierbehälter-Volumen (2) vollständig ausgefüllt ist, und
d) Durchleiten einer definierten Menge des Mediums (4, 5, 6) durch das mit dem Odoriermedium (12) befüllten Dosierbehälter-Volumen (2).

2. Verfahren nach Anspruch 1, **dadurch gekennzeichnet; dass** der Dosierbehälter (1) auf einen Druck zwischen 10 und 20 mbar evakuiert wird.

3. Verfahren nach Anspruch 1 oder 2, **dadurch gekennzeichnet, dass** das während des Verfahrensschrittes b) aus dem Dosierbehälter (1) abgezogene Gemisch adsorptiv von dem Odoriermedium befreit wird.

4. Verfahren nach einem der vorhergehenden Ansprüche 1 bis 3, **dadurch gekennzeichnet, dass** das Medium Kohlendioxid ist.

## Claims

1. Method for controlled admixing of an odorizing medium to a medium, the odorizing medium and the medium being intermixed in a metering container (1) of variable volume (2), comprising the following method steps:
a) setting of a defined metering container volume (2),
b) evacuation of the metering container (1),
c) introduction of a defined quantity of the odorizing medium (12) into the metering container (1), the introduction of the defined quantity of the odorizing medium (12) being continued until the metering container volume (2) is filled completely, and
d) passage of a defined quantity of the medium (4, 5, 6) through the metering container (2) filled with the odorizing medium (12).

2. Method according to Claim 1, **characterized in that** the metering container (1) is evacuated to a pressure of between 10 and 20 mbar.

3. Method according to Claim 1 or 2, **characterized in that** the mixture drawn off from the metering container (1) during method step b) is freed of the odorizing medium by adsorption.

4. Method according to one of the preceding Claims 1 to 3, **characterized in that** the medium is carbon dioxide.

## Revendications

1. Procédé de mélange contrôlé d'un milieu odorant dans un milieu, le milieu odorant et le milieu étant mélangés dans un contenant d'alimentation (1) modifiable au regard de son volume (2), comprenant les étapes de procédé suivantes :
a) l'ajustement d'un volume (2) défini du contenant d'alimentation,
b) l'évacuation du contenant d'alimentation (1),
c) l'introduction d'une quantité définie du milieu odorant (12) dans le contenant d'alimentation (1), l'introduction de la quantité définie du milieu odorant (12) étant poursuivie jusqu'à ce que le volume (2) du contenant d'alimentation soit complètement rempli, et
d) le passage d'une quantité définie du milieu (4, 5, 6) au travers du volume (2) du contenant d'alimentation rempli avec le milieu odorant (12).

2. Procédé selon la revendication 1, **caractérisé en ce que** le contenant d'alimentation (1) est évacué à une pression comprise entre 10 et 20 mbar.

3. Procédé selon la revendication 1 ou 2, **caractérisé en ce que** le mélange soutiré du contenant d'alimentation (1) pendant l'étape de procédé b) est débarrassé par adsorption du milieu odorant.

4. Procédé selon l'une quelconque des revendications 1 à 3 précédentes, **caractérisé en ce que** le milieu est le dioxyde de carbone.
